# EUROPEAN PATENT APPLICATION

(11) **EP 4 193 936 A1**
(43) Date of publication of application: **14.06.2023**
(21) Application number: 22212140.2
(22) Date of filing: 08.12.2022
(51) Int. Cl.: A61B 17/12

(54) **IMPLANTABLE MEDICAL DEVICE DETACHMENT SYSTEM WITH RETRACTABLE MECHANICAL RELEASE MECHANISM**

(30) Priority: 09.12.2021 US 202117546121
(71) Applicant: DePuy Synthes Products, Inc., Raynham, MA 02767 (US)
(72) Inventor: SOLAUN, Daniel, Raynham, 02767 (US); STEFANOV, Petrica, Raynham, 02767 (US); ALES, Francisco, Raynham, 02767 (US); DICKERSON, Jasia, Raynham, 02767 (US)
(74) Representative: Carpmaels & Ransford LLP

(57) **Abstract**

A detachment system configured to deliver an implantable medical device to a target location of a body vessel includes a generally hollow distal tube and an engagement system. The distal tube can have an inner lumen having a first diameter therethrough and a narrowed portion positioned on at least a portion of the distal tube and having a second diameter lesser than the first diameter. The engagement system can be slidably disposed within the inner lumen and have a detachment feature connected to the implantable medical device and a locking member engaging the detachment feature and interfacing with the narrowed portion of the distal tube. The locking member is configured to deploy the implantable medical device when the locking member is displaced from the narrowed portion of the distal tube.

## Description

### FIELD OF THE INVENTION

The present invention relates to a medical device for placing an implantable medical device at a predetermined site within a vessel of the human body, and more particularly, relates to a catheter-based deployment system for delivering an embolic device.

### BACKGROUND

For many years, flexible catheters have been used to place various devices within the vessels of the human body. Such devices include dilation balloons, radiopaque fluids, liquid medications, and various types of occlusion devices such as balloons and embolic coils. Coils which are placed in vessels may take the form of helically wound coils, or alternatively, may take the form of randomly wound coils, coils wound within coils or other such coil configurations. Often, several coils are placed at a given location to occlude the flow of blood through the vessel, or aneurysm, by promoting thrombus formation at the particular site.

In the past, embolic coils have been placed within the distal end of a catheter. When the distal end of the catheter is properly positioned, the coil may then be pushed out of the end of the catheter with a pusher member to release the coil at the desired location. This procedure for placement of an embolic coil is conducted under fluoroscopic visualization such that the movement of the coil through the vasculature of the body may be monitored and the coil placed at the desired location.

Other coil deployment systems incorporate interlocking mechanisms on the coil. In such systems, the interlocking end on the embolic coil couples with a similar interlocking mechanism on a pusher assembly. In one example system, a control wire extends through the locking mechanism and secures the coil to the pusher assembly. When the embolic coil is pushed out of the end of the catheter for placement, the control wire is retracted, and the coil disengages from the pusher assembly. Such a deployment system is disclosed in U.S. Pat. No. 5,925,059, entitled "Detachable Embolic Coil Assembly."

Another system includes a catheter with an expandable reaction chamber within the catheter lumen and a gripper at the distal end of the catheter that is lassoed around a headpiece of a coil. The reaction chamber has multiple chambers separated by a heat-dissolvable membrane. When the membrane is dissolve, the components from the chambers react and expand, causing the expandable gripper to deploy the coil. Such a coil positioning system is disclosed in U.S. Pat. No. 8,449,591, entitled "Chemically based vascular occlusion device deployment."

Yet another coil deployment system is that of a catheter having a locking member with a Y-shaped hook holding a loop of the coil at the distal end of the catheter. When the Y-shaped hook is deployed out of the catheter sleeve, the hook opens and thus releases the loop and deploys the coil. Such coil deployment system is disclosed in U.S. Pat. No. 8,974,488, entitled "Delivery assembly for occlusion device using mechanical interlocking coupling mechanism."

Still another system for placing an embolic coil within a vessel is that of a plunger device with an enlarged head within a catheter which can be pulled against tapered internal surfaces of the catheter, causing the gripper arms of the catheter to expand and release the coil at the end of the gripper arms. Such a procedure is disclosed in U.S. Pat. No. 10,034,670, entitled "Medical implant detachment mechanism and introducer assembly."

Yet another coil deployment system incorporates a catheter having a pushrod with a flexible socket at the distal end holding a ball-tip of a coil. When the pushrod is advanced distally such that the flexible socket is outside of the catheter, the socket deforms to allow the ball-tip to be released and thereby deploy the coil. Such a coil positioning system is disclosed in U.S. Pat. No. 10,888,331, entitled "Active release of embolic coils."

However, for at least some of the above systems, it can be difficult to positively and smoothly releasing the embolic coil. Applicants recognize that there is a need to securely hold the pull-wire in place until the embolic coil is ready to be deployed to avoid premature detachment, however, if the securement force is too high, the embolic coil may not be easily released. Thus, there is a need for alternative systems which accomplish these goals.

### SUMMARY

Thus, an example of an implantable medical device detachment system to place an implantable medical device, such as an embolic coil or a balloon, at a predetermined site within a vessel can include a generally hollow distal tube and an engagement system. The distal tube can include a proximal end, a distal end, an inner lumen with a first diameter therethrough, and a narrowed portion with a second diameter less than the first diameter. The narrowed portion can be positioned on at least a portion of the distal end of the distal tube. The engagement system can be slidably disposed within the inner lumen. The engagement system can include a detachment feature and a locking member. The detachment feature can be connected to the implantable medical device. The locking member can include a proximal portion extending through the proximal end of the distal tube. Further, the locking member can include a distal end that can interface with the narrowed portion of the distal tube and engage the detachment feature. The locking member can also be configured to deploy the implantable medical device engaged at the distal end of the distal tube. Therefore, when the locking member is engaged with the detachment feature and the detachment feature is connected to the implantable medical device, the locking member is in an engaged position, retaining the implantable medical device proximal to the tip of the catheter. When the proximal portion of the locking member is pulled proximally, the locking member is displaced from the detachment feature, thereby releasing the implantable medical device. The implantable medical device can be an embolic coil.

The narrowed portion of the distal tube can include one or more protrusions. In some examples, the one or more protrusions can approximately circumscribe the inner lumen.

The locking member of the engagement system can further include two or more appendages. The appendages can be configured to move between an engaged configuration and an open configuration, and further define a cavity between the two or more appendages. The cavity can be configured to fit at least a portion of the detachment feature when the locking member is in the engaged configuration.

The locking member can be configured to deploy the detachment feature from the cavity when the two or more appendages are in the open configuration. In some examples, this is accomplished because the locking member includes a flexible material. The two or more appendages are in the engaged configuration when the locking member interfaces with the narrowed portion of the distal tube, and in the open configuration when the locking member is displaced from the narrowed portion of the distal tube. In some examples, when the locking member is moved proximally in a way that the distal end of the locking member is displaced from the narrowed portion of the distal tube, the locking member is configured to deploy the implantable medical device engaged at the distal end of the distal tube.

The locking member can additionally include a knob at a distal end of each respective appendage. The cavity of the locking member can include a cavity width. Further, the knobs on each of the one or more appendages can define a knob width. In some examples, the cavity width can be larger than the knob width when in the engaged configuration. Further, the detachment feature can include a detachment feature width. The detachment feature width can be sized between the cavity width and the knob width in the engaged configuration. When in the open configuration, the locking member can have an open locking member width formed by the knobs of each of the one or more appendages. The open locking member width can be larger than the knob width in the engaged configuration. The detachment feature can have a detachment feature width that is sized less than the open locking member width when in the open configuration.

The detachment system can further include a distal stopped positioned at the distal end of the distal tube.

Another example of detachment system similar to the above can include the detachment feature engaging with the locking member, where the locking member can be configured to be in the engaged configuration when interfacing with the narrowed portion of the distal tube and in the open configuration when displaced from the narrowed portion of the distal tube, thereby deploying the implantable medical device connected to the detachment feature. Additionally, the locking member can be configured to be pulled proximally through the distal tube such that the locking member is displaced from the narrowed portion. In some examples, this is accomplished because the locking member includes a flexible material, such as nitinol.

A method of manufacturing an example detachment system as described above can include narrowed at least a portion of a distal tube and positioning an engagement system within the narrowed portion of the distal tube. The engagement system can include a detachment feature connected to an implantable medical device and a locking member. The locking member can include a proximal portion extending through the distal tube, and a distal end having two or more flexible appendages defining a cavity configured to fit at least a portion of the detachment feature. The distal end can be configured to interface with the narrowed portion of the distal tube, engage the detachment feature, and deploy the implantable medical device engaged at a distal end of the distal tube.

Narrowing the distal tube can include crimping the distal tube in such a way that decreases a diameter of an inner lumen of the distal tube. Alternatively, or in addition thereto, narrowing the distal tube can include positioning one or more protrusions within the distal tube in such a way that decreases a diameter of an inner lumen of the distal tube.

The method can additionally include securing the detachment feature within the cavity formed from the two or more flexible appendages of the locking member in an engaged configuration. Further, the method can also include releasing the detachment feature from the cavity formed from the two or more flexible appendages of the locking member in an open configuration.

In some examples, the method includes sliding the locking member proximally within the distal tube such that upon displacement of the locking member from the narrowed portion, the locking member transitions from the engaged configuration to the open configuration.

### BRIEF DESCRIPTION OF THE DRAWINGS

This invention is described with particularity in the appended claims. The above and further aspects of this invention may be better understood by referring to the following description in conjunction with the accompanying drawings, in which like numerals indicate like structural elements and features in various figures. The drawings are not necessarily to scale, emphasis instead being placed upon illustrating the principles of the invention.

The drawing figures depict one or more implementations in accord with the present teachings, by way of example only, not by way of limitation. In the figures, like reference numerals refer to the same or similar elements.
FIGs. 1A through 1C show sectional side views of an example implantable medical device detachment system in accordance with the present invention;
FIGs. 2A through 2C illustrate cross-sectional end views of the inner lumen of FIG. 1A through FIG. 1C, respectively;
FIGs. 3A and 3B show sectional side views, illustrating various protrusions of an example detachment system;
FIGs. 3C and 3D show cross-sectional end views of the inner lumen of FIGs. 3A and 3B, illustrating various protrusions of an example detachment system;
FIGs. 4A and 4B are side views of an example locking member in the engaged configuration shown in solid lines and the open configuration in dotted lines;
FIG. 5A shows a perspective partial sectional view of an example implantable medical device detachment system illustrating the locking member engaging with the narrowed portion of the distal tube;
FIGs. 5B and 5C are cross-sectional end views of FIG. 5A illustrating locking members having two or more appendages;
FIG. 5D shows a perspective partial sectional view of an example implantable medical device detachment system illustrating the locking member slidably displaced from the narrowed portion of the distal tube;
FIGs. 5E and 5F are cross-sectional end views of FIG. 5D illustrating locking members having two or more appendages;
FIG. 6A is a perspective partial sectional view of an example implantable medical device detachment system illustrating a crimped narrowed portion;
FIG. 6B is a cross-sectional end view of FIG. 6A; and
FIG. 7 is a flow chart of the method of manufacturing an implantable medical device detachment system of the present invention.

### DETAILED DESCRIPTION

The following detailed description should be read with reference to the drawings, in which like elements in different drawings are identically numbered. The drawings, which are not necessarily to scale, depict selected examples and are not intended to limit the scope of the invention. The detailed description illustrates by way of example, not by way of limitation, the principles of the invention. This description will clearly enable one skilled in the art to make and use the invention, and describes several examples, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator whereas "distal" indicates a location further away to the operator or physician.

The figures illustrate a generally hollow or tubular structure according to the present invention. When used herein, the terms "tubular" and "tube" are to be construed broadly and are not limited to a structure that is a right cylinder or strictly circumferential in cross-section or of a uniform cross-section throughout its length. For example, the tubular structure or system is generally illustrated as a substantially right cylindrical structure. However, the tubular system may have a tapered or curved outer surface without departing from the scope of the present invention.

In a mechanical detachment system, a pull-wire holds the implantable medical device to the system and when the pull-wire with an engagement system described herein is translated in the proximal direction, the engagement system detaches the implant to the target location. It is crucial to hold the wire, engagement system, and implant in place until it is ready to be translated. Inability to hold the wire in place may cause a premature detachment of the implant. A detachment system with a reliably secured locking member and readily slidable detachment mechanism may avoid premature detachment as well as an overly high securement force required for deployment. The pull-wire may have a solid or hollow core along a majority of its length.

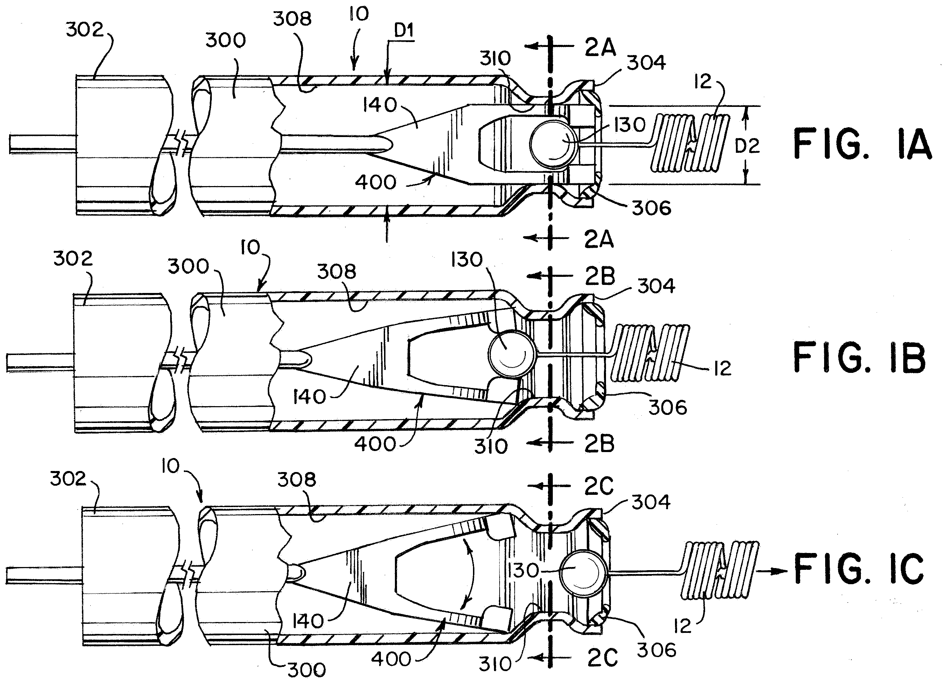

An example of a detachment system 10 of the present invention, as illustrated in FIGs. 1A through 1C, can have a generally hollow distal tube 300 and an engagement system 400. The distal tube 300 can have a proximal end 302, a distal end 304, an inner lumen 308 with a first diameter D1 therethrough, and a narrowed portion 310 positioned on at least a portion of the distal end 304. The inner lumen 308 of the narrowed portion 310 can have a second diameter D2 that is sized less than the first diameter D1. An implantable medical device 12 is engaged at one end of the distal tube 300. The implantable medical device 12 can be an embolic coil, but it can be appreciated that virtually any implantable medical device 12 may be delivered and deployed by the detachment system 10 according to the present invention. The medical device 12 is connected to a detachment feature 130, which is, in turn, is engaged to a locking member 140. At least a portion of the distal tube 300 has a narrowed lumen such that the narrowed portion interfaces with the locking member 140 to maintain an engaged configuration of the locking member 140, as illustrated in FIG. 1A. As the locking member 140 is displaced from the narrowed portion 310 of the distal tube 300, as depicted in FIG. 1C, the locking member 140 transitions to an open configuration, and thus deploys the detachment feature 130 and medical device 12. In some examples, as shown in FIGs. 1A through 1C, the distal tube 300 can also include a distal stopper 306 positioned proximate an exit of the distal tube 300. The distal stopper 306 can be sized to engage with at least a portion of the engagement system 400 such that the detachment feature 130 can smoothly exit the distal tube 300 during deployment, while maintaining the locking member 140 within the distal tube 300 before, during and after deployment of the implantable medical device 12.

The detachment system 10 may include a medical device 12 which, in an example, takes the form of a helically wound embolic coil disposed at the distal end 304 of the distal tube 300. While the medical device 12 as is illustrated is shown as a helically wound coil, other types of embolic devices, such as filaments, braids, foams, expandable meshes and stents, could be delivered using the present deployment system and various other coil configurations could be delivered using this system. A coil may be relatively stiff and made of stainless steel or it may be soft and made of platinum. Extremely soft coils may be made with either a spiral shape or a more complex shape to promote deployment at the desired delivery location and to promote a higher packing density. The diameter of a coil is selected in consideration of the size of the aneurismal sac. Generally, the medical device 12 may be very small and thin, ranging in a variety of shapes and sizes. The medical device 12 may come in various random loop designs to conform to the aneurysm shape, and various deployments of the coil device may be used. A coil can vary in softness and in stiffness. The coil size can range from about twice the width of a human hair to less than one hair's width. The number of loops in a coil may vary. Platinum coils may be between 0.010 inches and 0.025 inches in diameter. A coil may vary from 1 to 60 centimeters in length, with some as long as 100 centimeters.

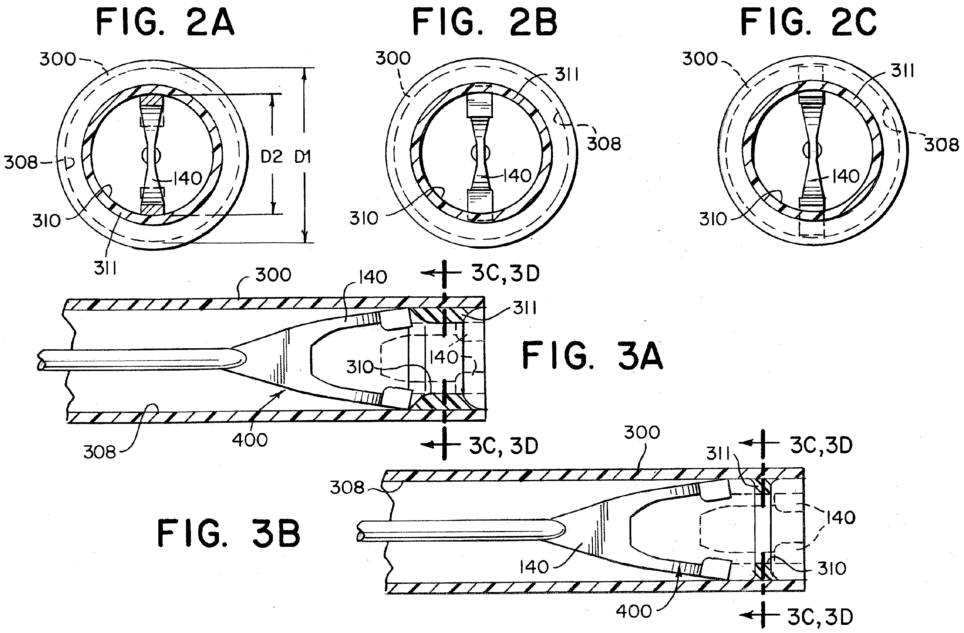

FIGs. 2A through 2C illustrate cross-sectional end views of the inner lumen 308 of the distal tube 300 of FIG. 1A through FIG. 1C, respectively. When the locking member 140 interfaces the smaller diameter D2 of the narrowed portion, the locking member 140 is in an engaged configuration, and although not depicted, the detachment feature 130 is gripped within the locking member 140. When the locking member 140 is slidably displaced from the narrowed portion, the locking member then expands to the larger diameter D1 of the inner lumen 308 of the distal tube 300 and thereby releases the detachment feature 130 and deploys the medical device 12.

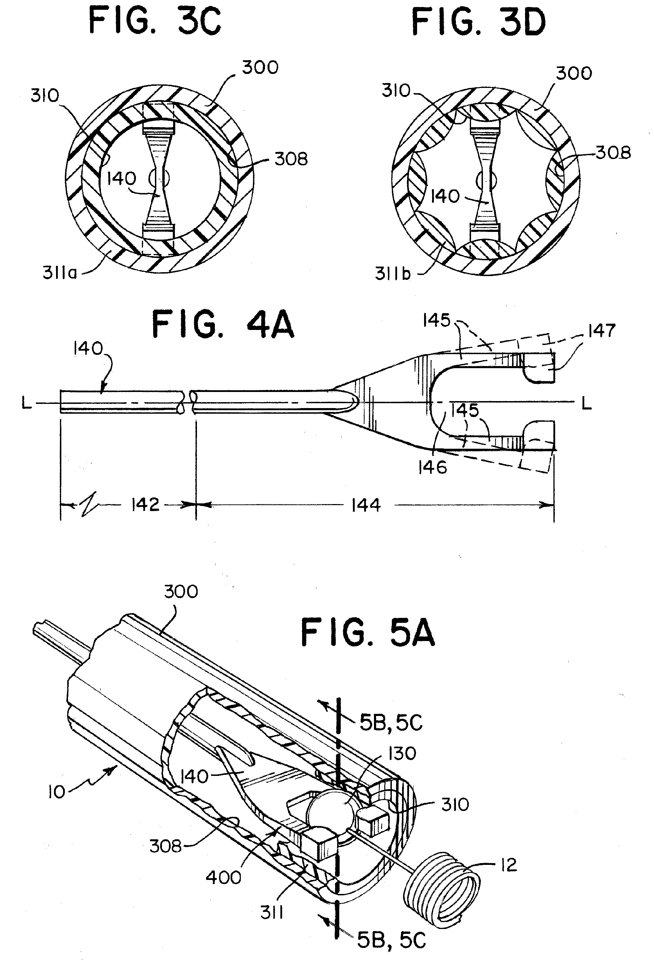

As shown in FIGs. 3A through 3D, the distal tube 300 can include a narrowed portion 310 having one or more protrusions 311a, 311b that cause the diameter D2 of the lumen of the narrowed portion 310 to be lesser than the diameter D1 of the inner lumen 308 of the remainder of the distal tube 300. The length of the narrowed portion 310 can be adjusted to increase or decrease the detachment mechanism securement force required for deployment. The narrowed portion 310 of the distal tube 300 can include a single circumferential protrusions 311a, as shown in FIG. 3C. Alternatively, or in addition thereto, the narrowed portion can include a series of protrusions 311b, as illustrated in FIG. 3D. In some aspects, the narrowed portion 310 can include protrusions having a variety of shapes or series of beveling.

The distal tube 300 can be made of a biocompatible material, such as stainless steel. The distal tube 300 can typically have a diameter of between about 0.010 inch and about 0.018 inch, a preferred tube having a diameter of approximately 0.0145 inch. These examples of tube size are suitable for delivering and deploying embolic coils to target locations, typically aneurysms, within the neurovasculature. Differently sized tubes 300 comprised of other materials may be useful for different applications and are within the scope of the present invention.

Although not depicted, in some examples, the distal tube 300 can be delivered to a subject using an outer catheter. The catheter size is selected in consideration of the size, shape, and directionality of the aneurysm or the body lumens the catheter must pass through to get to the treatment site. The catheter 2 may have a total usable length anywhere from 80 centimeters to 165 centimeters and a distal length of anywhere between 5 centimeters to 42 centimeters. The catheter 2 may have an inner diameter ID of anywhere between 0.015 and 0.025 inches. The outer diameter ID may also range in size and may narrowed at either its proximal end or distal end. The outer diameter may be 2.7 French or less.

The engagement system 400 can be slidably disposed within the inner lumen 308 of the distal tube 300. The engagement system 400 can include a detachment feature 130 connected to the implantable medical device and a locking member 140. The detachment feature 130 can be configured to fit inside the inner lumen 308 of the distal tube 300 and interface with the locking member 140. The detachment feature 130 can have a proximal coupling portion that is separate from the implantable medical device 12 such that the locking member 140 interfaces the proximal coupling portion of the detachment feature 130 within the inner lumen 108 of the distal tube 300 while the implantable medical device 12 remains distal and external of the distal tube 300.

FIG. 4A is a plan view of an example locking member 140 in the engaged configuration shown in solid lines and the open configuration in dotted lines. The locking member 140 can serve to interlock the detachment feature 130 and implantable medical device 12 at the distal end 304 of the distal tube 300 until such time as the locking member 140 is withdrawn proximally and transitions from the engaged configuration to the open configuration. As shown, the locking member 140 can have a proximal portion 142 and a distal end 144. The locking member 140 can extend through the proximal end 302 of the distal tube 300 along a longitudinal axis L-L toward the operator or medical professional. The locking member 140 can be attached to a surgical device at its proximal end. The locking member 140 preferably takes the form of a small diameter elongate filament, however, other forms such as wires or tubular structures are also suitable. The proximal portion 142 of the locking member 140 can be relatively small, having the thickness of a hair in some examples, so it may be preferred for it to be entirely shielded by the distal end 304 of the distal tube 300 to prevent damage from accidental contact. The proximal portion 142 of the locking member 140 can be an elongated wire or a flat ribbon formed from of any of a number of materials, including nitinol and stainless steel.

As depicted in FIGs. 1A through 1C, the distal end 144 of the locking member 140 can be positioned near the distal end 304 of the distal tube 300. The locking member 140 interfaces with the narrowed portion 310 of the distal tube 300 and engages with the detachment feature 130. The locking member 140 can be slidably disposed within the inner lumen 308 of the distal tube 300 such that when the locking member 140 interfaces with the narrowed portion 310, the detachment feature 130, and by attachment, the medical device 12 remains engaged with the distal tube 300. When the locking member 140 is slidably displaced from the narrowed portion, the locking member 140 disengages the detachment feature and deploys the medical device 12. The locking member 140 in combination with the coupling portion of the detachment feature 130 provides a mechanism by which the medical device 12 can be gripped in the engaged configuration but released in the open configuration.

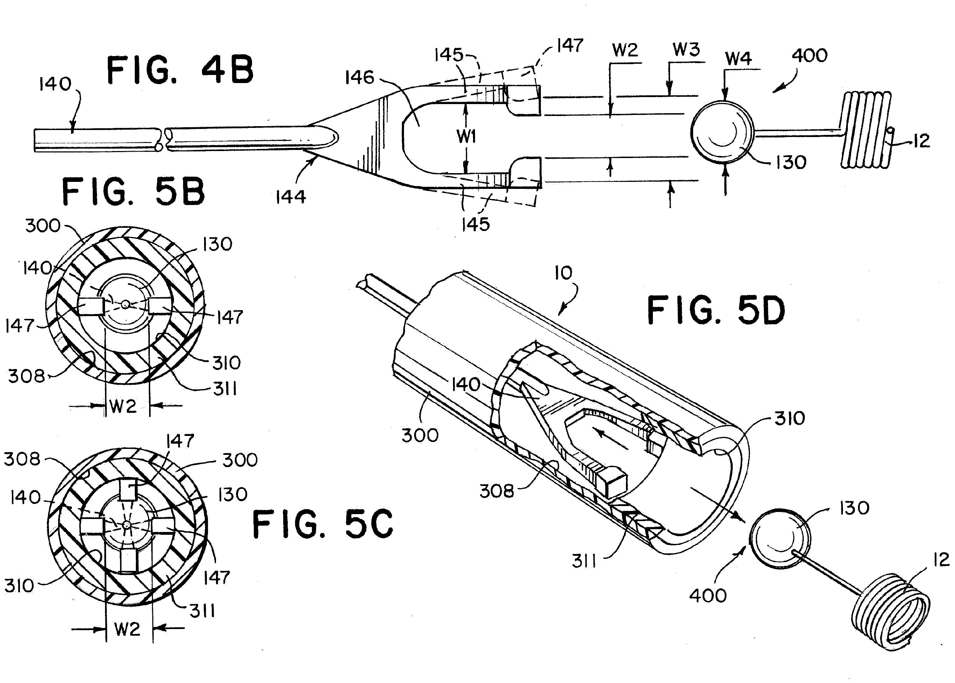

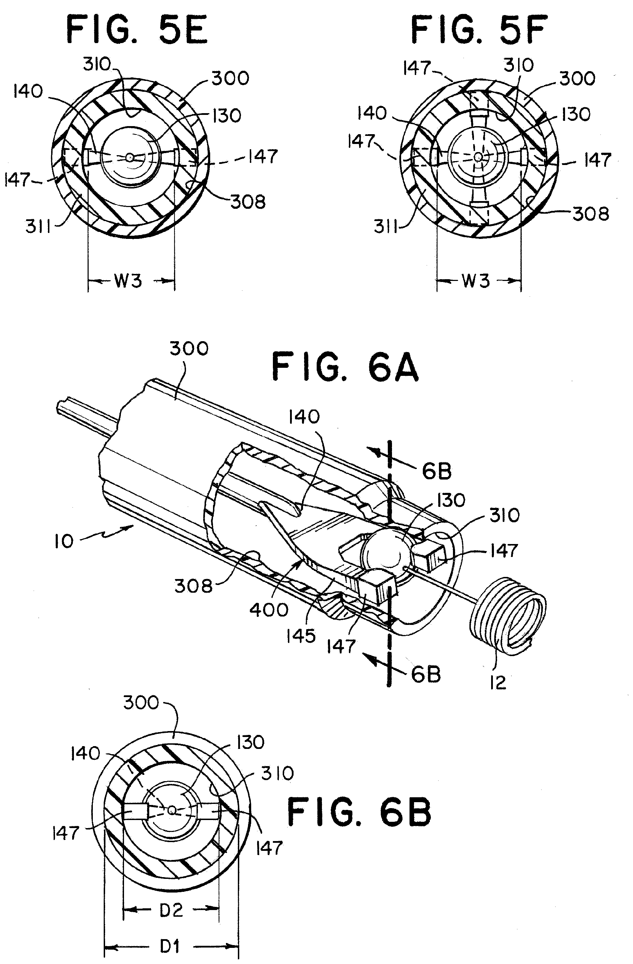

The distal end 144 of the locking member 140, as shown in FIGs. 4A and 4B, can include two appendages 145 configured to move between an engaged configuration and an open configuration. In certain examples, the locking member 140 can include four appendages 145, as illustrated in FIGs. 5C and 5F, although fewer or more appendages are also contemplated (e.g., three, five, six, or more appendages). The appendages 145 can be made of a flexible material, such as nitinol. While the locking member 140 is preferably formed of nitinol, other metals and materials such as stainless steel, PTFE, nylon, ceramic or glass fiber and composites may also be suitable. The appendages 145 can be elastically deformable to the up-turned or open condition such that each appendage will return to the substantially open configuration when not otherwise constrained. In some examples, the appendages 145 can rely on static forces to engage with the detachment feature 130. In addition, or alternatively thereto, the locking member 140 can further include a knob 147 positioned at or near the distal end of each respective appendage 145 to assist in engaging the detachment feature 130 within the appendages 145 of the locking member 140.

As depicted in FIG. 4B, the two or more appendages 145 define a cavity 146 having a cavity width W1. At the distal end of each appendage 145, the knobs 147 can define a knob width W2 that is smaller than the cavity width when in the engaged configuration. When in the open configuration, the appendages 145 in the open configuration can define an open locking member width W3. The coupling portion of the detachment feature 130 can have a detachment feature width W4 that is sized between the cavity width W1 and the knob width W2 when the locking member 140 is in the engaged configuration, such that the detachment feature 130 is configured to securely fit within the cavity 146 of the locking member 140. When in the open configuration, the detachment feature width W4 is smaller than both the cavity width W1 and the open locking member width W3, such that the detachment feature 130 is not secure within the cavity 146 but is released and thereby deploy the medical device 12.

FIG. 5A shows a partial sectional view of an example implantable medical device detachment system 10 illustrating the locking member 140 engaging with the narrowed portion 310 of the distal tube 300 and thereby securing detachment feature 130 and implantable medical device 12, while FIG. 5D depicts the locking member 140 in the open configuration when the locking member 140 is withdrawn proximally and displaced from the narrowed portion 310, thereby releasing the detachment feature 130. FIGs. 5B and 5C show cross-sectional views of FIG. 5A with the locking member 140 in the engaged configuration. When in the engaged configuration, the two or more appendages 145 and the respective knobs 147 can form a knob width W2 that is smaller than the width of the detachment feature 130, thereby maintaining or securing the detachment feature 130 within the inner lumen 308 of the distal tube 300 prior to displacement of the locking member 140 from the narrowed portion 310. FIGs. 5E and 5F show cross-sectional views of FIG. 5D with the locking member 140 in the open configuration. When in the open configuration, the two or more appendages 145 and the respective knobs 147 can form an open locking member width W3 that is larger than the width of the detachment feature 130, thereby allowing the detachment feature 130 to escape the inner lumen 308 of the distal tube 300 when the locking member 140 is displaced from the narrowed portion 310. As shown, the locking member 140 can have two appendages 145 (FIG. 5B and 5E) or four appendages 145 (FIG. 5C and 5F), however the number of appendages 145 are to be construed broadly and are not limited to the number and shapes of appendages shown.

FIG. 6A is a partial sectional view of an example implantable medical device detachment system 10 illustrating a crimped narrowed portion 310. In some examples, instead of one or more protrusions or in addition thereto, the distal tube 300 can be crimped to form the narrowed portion 310 out of the same continuous material as the distal tube. As described above, the locking member 140 can then engage with the crimped narrowed portion 310 to maintain the engaged configuration around the detachment feature 130. As would be appreciated by one of skill in the art, additional or alternative methods to create a narrowed portion 310 can be implemented for the detachment system 10 described herein.

FIG. 6B shows a cross-sectional view of FIG. 6A with the distal tube 300 having an inner lumen 308 having a first diameter D1 and a crimped narrowed portion 310 having a second diameter D2 that is smaller than the first diameter Dl. As previously described, the locking member 140 can be in the engaged configuration with the detachment feature 130 maintained within the inner lumen 308 of the distal tube 300 prior to displacing the locking member 140 from the crimped narrowed portion 310.

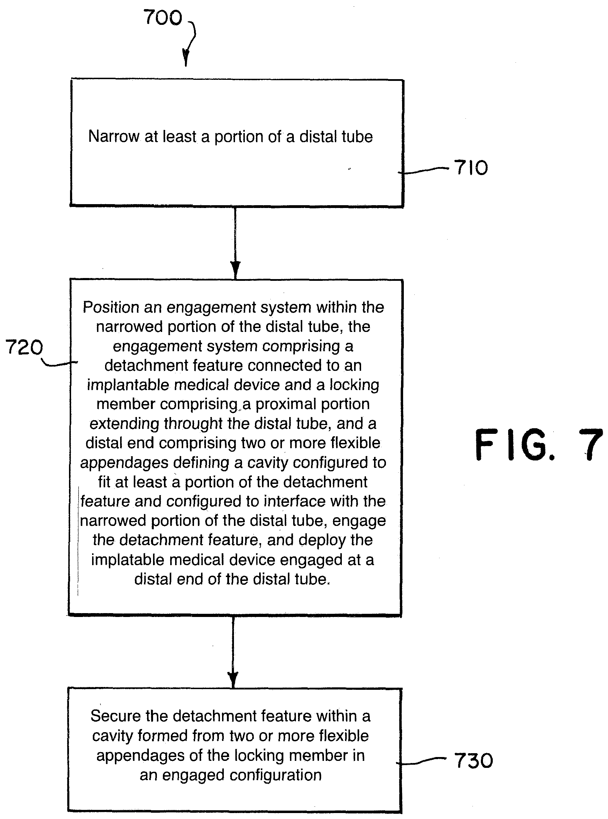

FIG. 7 is a flow chart of the method 700 for manufacturing an implantable medical device detachment system of the present invention. Method 700 can include narrowing 710 at least a portion of a distal tube. Narrowing 710 can include crimping the distal tube in such a way that decreases the diameter of the inner lumen at the narrowed portion of the distal tube. In some examples, narrowing 710 can include positioning one or more protrusions within the distal tube so as to decrease the diameter. Method 700 can further include positioning 720 an engagement system within the distal tube, and preferably within the narrowed portion of the distal tube. The engagement system can include a detachment feature connected to an implantable medical device and a locking member having a proximal portion extending through the distal tube, and a distal end configured to interface with the narrowed portion of the distal tube, engage the detachment feature, and deploy the implantable medical device engaged at a distal end of the distal tube. Method 700 can further include securing 730 the detachment feature within the cavity formed from the two or more flexible appendages of the locking member in an engaged configuration. Method 700 can end after securing 730 the detachment feature within the cavity of the locking member or can further include sliding the locking member proximally within the distal tube such that upon displacement of the locking member from the narrowed portion. In any of the examples described herein, the locking member can transition from the engaged configuration to the open configuration, and thereby release the detachment feature from the cavity formed from the two or more flexible appendages of the locking member in the open configuration.

As is apparent, there are numerous modifications of the preferred example described above which will be readily apparent to one skilled in the art, such as many variations and modifications of the embolic device including numerous coil winding configurations, or alternatively other types of embolic devices. Also, there are many possible variations in the materials and configurations of the release mechanism. These modifications would be apparent to those having ordinary skill in the art to which this invention relates and are intended to be within the scope of the claims which follow.

## Claims

1. A detachment system for delivering an implantable medical device to a target location of a body vessel, the detachment system comprising:
a generally hollow distal tube comprising a proximal end, a distal end, an inner lumen comprising a first diameter therethrough, and a narrowed portion positioned on at least a portion of the distal end and comprising a second diameter, the second diameter less than the first diameter; and
an engagement system slidably disposed within the inner lumen, the engagement system comprising:
a detachment feature connected to the implantable medical device; and
a locking member comprising:
a proximal portion extending through the proximal end of the distal tube, and
a distal end interfacing with the narrowed portion of the distal tube, engaging the detachment feature, and configured to deploy the implantable medical device engaged at the distal end of the distal tube.

2. The detachment system of claim 1, wherein the narrowed portion comprises one or more protrusions approximately circumscribing the inner lumen.

3. The detachment system of claim 1, wherein the locking member comprises:
two or more appendages configured to move between an engaged configuration and an open configuration and defining a cavity positioned between the two or more appendages, the cavity configured to fit at least a portion of the detachment feature when in the engaged configuration.

4. The detachment system of claim 3, wherein the locking member is further configured to deploy the detachment feature from the cavity when the two or more appendages are in the open configuration.

5. The detachment system of claim 3, wherein the locking member further comprises a flexible material, or a knob at a distal end of each respective appendage.

6. The detachment system of claim 3, wherein
the two or more appendages are in the engaged configuration when the locking member interfaces with the narrowed portion, and
the two or more appendages are in the open configuration when the locking member is displaced from the narrowed portion.

7. The detachment system of claim 1, wherein
the locking member is configured to deploy the implantable medical device engaged at the distal end of the distal tube when moved proximally such that the distal end of the locking member is displaced from the narrowed portion.

8. The detachment system of claim 3 wherein the locking member further comprises the know at the distal end of each respective appendage, wherein
the cavity of the locking member comprises a cavity width larger than a knob width formed by the knobs of the one or more appendages in the engaged configuration, and
the detachment feature comprises a detachment feature width between the cavity width and the knob width when in the engaged configuration.

9. The detachment system of claim 8, wherein
the locking member comprises an open locking member width formed by the knobs of the one or more appendages when in the open configuration, the open locking member width being larger than the knob width in the engaged configuration, and
the detachment feature comprises a detachment feature width less than the open locking member width when in the open configuration.

10. The detachment system of claim 1, further comprising a distal stopper (306) positioned at the distal end of the distal tube.

11. A detachment system for delivering an implantable medical device to a target location of a body vessel, the detachment system comprising:
a detachment feature connected to the implantable medical device; and
a locking member disposed within an inner lumen of a generally hollow distal tube, the locking member comprising:
a proximal portion extending through the inner lumen of the distal tube,
two or more appendages positioned at a distal end, having an engaged configuration and an open configuration, and
a cavity formed between the two or more appendages, the cavity being configured to secure the detachment feature when the two or more appendages are in the engaged configuration,
wherein the locking member is configured to be in the engaged configuration when interfacing with a narrowed portion of the distal tube and in the open configuration when displaced from the narrowed portion of the distal tube, thereby deploying the implantable medical device connected to the detachment feature, and
wherein the locking member is configured to be pulled proximally through the distal tube such that the locking member is displaced from the narrowed portion.

12. The detachment system of claim 11, wherein the narrowed portion comprises one or more protrusions approximately circumscribing the inner lumen.

13. The detachment system of claim 11, wherein the locking member comprises nitinol.

14. A method comprising:
narrowing at least a portion of a distal tube; and
positioning an engagement system within the narrowed portion of the distal tube,
the engagement system comprising:
a detachment feature connected to an implantable medical device; and
a locking member comprising:
a proximal portion extending through the distal tube, and
a distal end comprising two or more flexible appendages defining a cavity configured to fit at least a portion of the detachment feature, the distal end configured to interface with the narrowed portion of the distal tube, engage the detachment feature, and deploy the implantable medical device engaged at a distal end of the distal tube.

15. The method of claim 14, wherein:
narrowing the distal tube comprises (i) crimping the distal tube in such a way that decreases a diameter of an inner lumen of the distal tube, or (ii) positioning one or more protrusions within the distal tube in such a way that decreases a diameter of an inner lumen of the distal tube.

16. The method of claim 14, further comprising:
securing the detachment feature within the cavity formed from the two or more flexible appendages of the locking member in an engaged configuration, optionally further comprising: releasing the detachment feature from the cavity formed from the two or more flexible appendages of the locking member in an open configuration, further optionally comprising: sliding the locking member proximally within the distal tube such that upon displacement of the locking member from the narrowed portion, the locking member transitions from the engaged configuration to the open configuration.
